# EUROPEAN PATENT APPLICATION

(11) **EP 2 031 065 A1**
(43) Date of publication of application: **04.03.2009**
(21) Application number: 07115093.2
(22) Date of filing: 28.08.2007
(51) Int. Cl.: C12N 15/38, A61K 39/245, C12N 7/04

(54) **A recombinant koi herpesvirus (KHV) or Cyprinid herpesvirus 3 (CyHV-3) and a vaccine for the prevention of a disease caused by KHV/CyHV-3 in Cyprinus carpio carpio or Cyprinus carpio koi**

(71) Applicant: Université de Liège, 4031 Angleur (Liege) (BE)
(72) Inventor: Costes, Bérénice, Dr. University of Liège, 4000 Liège (BE); Lieffrig, François, Dr., 6900 Marloie (BE); Vanderplasschen, Alain, Dr. University of Liège, 4000 Liège (BE)
(74) Representative: Polypatent

(57) **Abstract**

The present invention refers to a recombinant koi herpesvirus (KHV) or Cyprinid herpesvirus 3 (CyHV-3), which is immunogenic in fish, preferably in carps, more preferably in *Cyprinus carpio,* and to a vaccine for preventive and/or therapeutic treatment of a disease caused by koi herpesvirus (KHV) or CyHV-3.

## Description

### Field of the invention

The present invention refers to a recombinant koi herpesvirus (KHV) or Cyprinid herpesvirus 3 (CyHV-3) and a vaccine for the prevention of a disease caused by koi herpesvirus/Cyprinid herpesvirus 3 in *Cyprinus carpio carpio* or *Cyprinus carpio koi.*

### Background of the invention

Common carp (*Cyprinus carpio carpio*) is the most widely cultivated fish for human consumption mainly in Asia, Europe, and the Middle East. In contrast, the koi (*Cyprinus carpio koi)* subspecies is cultivated as an expensive beautiful and colorful pet fish for personal pleasure or competitive showing especially in Japan but also worldwide. A virus causing a lethal disease in common and koi carps, initially called koi herpesvirus disease (KHVD), was detected in 1996 in the United Kingdom. The virus was then rapidly identified as the cause of mass mortality among koi and common carps in Israel, the USA, and Germany. Intensive culture of common carps, koi shows and international trading have unfortunately contributed to the rapid global spread of this highly contagious and extremely virulent disease. Since its emergence, KHVD has caused severe financial and economic losses in both koi and carp culture industries worldwide.

Initial characterization of the virus showed a herpes-like structure with an envelope and an icosahedral electron-dense core of 100-110 nm surrounded by a tegument-like structure. The genome of the virus comprises linear double-stranded DNA (dsDNA) of ∼295 kb similar to that of Cyprinid herpesvirus 1 (CyHV-1) but larger than those of other *Herpesviridae* members generally ranging from 125 to 240 kb in size. The sequence of KHV genome has been published very recently (Aoki et al., J Virol, 81, pages 5058-5065 (2007)). The KHV genome contains a significant number of DNA sequences without homology to any other known viral sequences. Moreover, it contains highly divergent DNA sequences encoding polypeptides, which resemble those of several dsDNA viruses, like herpesvirus, poxvirus, iridovirus and other large DNA viruses.

The unique characteristics of this virus have led to three different nomenclatures: firstly, koi herpesvirus (KHV) according to its morphological manifestation; secondly, carp interstitial nephritis and gill necrosis virus (CNGV) according to its pathogenic effects in fish; and lastly Cyprinid herpesvirus 3 (CyHV-3) according to gene content similarity with CyHV-1 and CyHV-2. The latter nomenclature has been further supported by the recent sequencing of the full length of the viral genome. However, hereinafter the name KHV will be used.

KHV bears a genome of approximately 295 kb which represents the largest genome ever identified among *Herpesviridae* members. Since the first isolation of KHV in 1996, no information is available about the role of individual genes in KHV pathogenesis and in the biology of the infection of the natural host.

An attenuated vaccine candidate has been described in the international patent application WO 2004/061093 A1. However, this vaccine candidate exhibits two major defaults. Firstly, the attenuation is the consequence of random mutations that occurred during viral replication *in vitro.* Consequently, the determinism of the attenuation is unknown and reversion to a fully pathogenic phenotype can not be excluded. Secondly, the vaccine strain exhibits residual virulence in small juvenile fishes killing up to 20% of vaccinated subjects.

### Problem and solution

Since its first isolation in 1996, an increasing number of studies have been devoted to KHV. They reported data related to viral gene content, to viral pathogenesis, to the epidemiology of the infection, to the diagnostic of the infection and to control methods. However, to date no KHV recombinant strain has been produced, and consequently, the role of individual genes in KHV pathogenesis and in the biology of infection of the natural host is unknown. Similarly, the absence of KHV recombinant strains can explain the lack of safe and efficacious attenuated recombinant vaccines for the control of the disease on the market.

Applied and fundamental researches on KHV require production of recombinant virus. Recently, manipulation of large herpesvirus genomes has been facilitated by the use of bacterial artificial chromosomes (BAC) vectors (Messerle et al., Proc Natl Acad Sci U S A, 94, 14759-14763 (1997); Wagner et al., Trends Microbiol, 10, 318-324 (2002)). These vectors allow the maintenance and efficient mutagenesis of the viral genome in *Escherichia coli (E.coli)* followed by the reconstitution of progeny virions by transfection of the BAC plasmid into permissive eukaryotic cells. To date, the genomes of several herpesviruses have been successfully propagated as infectious BAC clones, including Human Cytomegalovirus (HCMV) which represents the largest herpesvirus genome cloned as a BAC to date (230 Kb) (Borst et al., J Virol, 73, 8320-8329 (1999)).

There is a clear need for development of safe and efficacious attenuated vaccines to control KHV infection. These fundamental and applied researches on KHV require the production of KHV recombinant strains.

The object of the present invention therefore was to provide a strain or construct of KHV as vaccine which can be used for the development of safe and efficacious attenuated vaccines to control KHV infection.

The object was solved by a recombinant koi herpesvirus (KHV) or Cyprinid herpesvirus 3 (CyHV-3), which is immunogenic in fish, preferably in carps, more preferably in *Cyprinus carpio,* even further preferred in *Cyprinus carpio carpio* and/or *Cyprinus carpio koi.*

So far, different groups of scientists have attempted for several years to clone the KHV genome as a BAC and failed. They only obtained clones with a reduced genome. These problems may be due to the size of the KHV genome, which is larger than that of any other herpesvirus cloned so far: the largest herpesvirus cloned in the state of art has a size of 235 kb, while the KHV herpesvirus has a size in the range of 290 to 300 kb. Further problems for cloning of this virus were due to the many repetitive sequences comprised in the genome, which may result in unstable clones and reduced size. Therefore, the present invention provides for the first time a full-length infectious BAC clone of the KHV genome, having a size in the range of 290 to 300 kb. The present invention is also the first to produce attenuated recombinants for an herpesvirus genome having a size in the range of 290 to 300 kb.

The KHV BAC clone produced may be used for further manipulation involving for example genetic engineering techniques in order to make the genome deficient in specific genes. By that the size of the genome may strongly be decreased, in case of plurality of viral gene deletion. A derivative obtained therefore will still be within the scope of the present invention.

In a preferred embodiment the recombinant koi herpesvirus has the capability to confer immunity on fish, preferably on carps, more preferably on *Cyprinus carpio,* even further preferred on *Cyprinus carpio carpio* and/or *Cyprinus carpio koi,* against a disease caused by koi herpesvirus (KHV) or Cyprinid herpesvirus 3 (CyHV-3).

Preferably, the recombinant koi herpesvirus is deficient in one or more viral genes which is/are not essential for replication. More preferred the recombinant koi herpesvirus is deficient in at least one gene which contributes to virulence. It is particularly preferred that the recombinant koi herpesvirus according to the present invention is deficient in at least one gene which contributes to virulence selected from the group consisting of thymidine kinase gene; ORF12: putative tumor necrosis factor (TNF) receptor gene; ORF 16: putative G-protein coupled receptor (GPCR) gene; ORF 134: putative Interleukin 10 homologue gene; ORF140: putative thymidylate kinase gene, or any combination thereof. It is further preferred that the recombinant koi herpesvirus according to the present invention is deficient in viral thymidine kinase gene and at least one further gene which contributes to virulence selected from the group consisting of ORF12: putative tumor necrosis factor (TNF) receptor gene; ORF 16: putative G-protein coupled receptor (GPCR) gene; ORF 134: putative Interleukin 10 homologue gene; ORF140: putative thymidylate kinase gene, or any combination thereof. It is further particularly preferred that the recombinant koi herpesvirus is deficient in all of above mentioned genes. Moreover, the recombinant koi herpesvirus may be deficient in further viral genes.

In a further preferred embodiment of the present invention the recombinant koi herpesvirus is in a live form. Preferably, the recombinant koi herpesvirus has the capability to reconstitute infectious particles when introduced into permissive eukaryotic cells or fish individuals, preferably in carps, more preferably in *Cyprinus carpio,* even further preferred in *Cyprinus carpio carpio* and/or *Cyprinus carpio koi.*

Further preferred, the recombinant koi herpesvirus is in an attenuated form.

In a further preferred embodiment of the present invention the recombinant koi herpesvirus results in a mortality rate of 40 % or less, preferably of 20 % or less, more preferred of 10 % or less, further preferred of 1 % or less and most preferred of 0.1 % or less in fish, preferably in carps, more preferred *Cyprinus carpio carpio* or *Cyprinus carpio koi* infected with said herpesvirus.

In an alternative embodiment of the present invention the recombinant koi herpesvirus is in inactivated and non-replicative form or in deficient attenuated form. "Deficient attenuated form" means that the recombinant koi herpesvirus has the capability to infect cells or fish individuals (e.g. *Cyprinus carpio, Cyprinus carpio carpio* or *Cyprinus carpio koi)* but is not able to replicate.

Further preferred, the recombinant koi herpesvirus according to the present invention comprises a BAC (bacterial artificial chromosome) vector sequence. Preferably, the recombinant koi herpesvirus comprises a BAC (bacterial artificial chromosome) vector sequence which is inserted into the koi herpesvirus genome.

Further preferred, the recombinant koi herpesvirus comprises a BAC (bacterial artificial chromosome) vector sequence which is inserted into the viral thymidine kinase gene, or alternatively any other viral gene which contributes to virulence and/or any other viral gene which is not essential for viral replication. It is particularly preferred that the BAC vector sequence is flanked by sequences mediating homologous recombination, preferably *loxP.* Further preferred, the BAC vector sequence comprises a selectable marker. Even further preferred, the selectable marker is a drug selectable marker. Even more preferred the selectable marker is a gene encoding green fluorescent protein. In a further preferred embodiment the genome of said recombinant herpesvirus is present in the form of a plasmid. This is achieved by isolating circular forms of the above mentioned recombinant koi herpesvirus comprising a BAC vector sequence and introduction into bacterial cells.

In an alternative embodiment of the present invention the BAC vector sequence is excised from the herpesvirus genome thereby leaving a heterologous sequence at the excision site or former insertion site, respectively, in the herpesvirus genome. Preferably, the heterologous sequence has a size of less than 200 nucleotides. The excision is achieved by introduction of the recombinant KHV into a permissive eukaryotic cell expressing the Cre recombinase which is excising the *loxP*-flanked BAC vector sequence. The resulting recombinant KHV may preferably be able to form infectious particles.

In further preferred embodiments of the recombinant koi herpesvirus further viral genes are modified in their activity or deficient or deleted.

The present invention also provides a DNA sequence to be used for vaccine purposes and/or prophylactic health care in fish, wherein said DNA sequence which has the capability to confer immunity on fish, preferably on carps, more preferably on *Cyprinus carpio,* against a disease caused by koi herpesvirus (KHV) or Cyprinid herpesvirus 3 (CyHV-3).

The present invention also provides a vector comprising a koi herpesvirus genome or a part thereof. Preferably, the vector is further comprising a BAC vector sequence. Further preferred the vector has the capability to replicate and further preferred has the capability also to reconstitute infectious particles when introduced into permissive eukaryotic cells.

In a further preferred embodiment the vector is able to express KHV viral genes when introduced into permissive cells or fish individuals, in order to induce a protective immune response against a disease caused by koi herpesvirus (KHV) or Cyprinid herpesvirus 3 (CyHV-3). Further preferred, the vector has the capability to confer immunity on fish, preferably on carps, more preferably on *Cyprinus carpio,* against a disease caused by koi herpesvirus (KHV) or Cyprinid herpesvirus 3 (CyHV-3).

The present invention also provides a cell which comprises the recombinant koi herpesvirus or the DNA sequence or the vector according to the present invention as mentioned and characterized above. Preferably, the cell is a bacterial cell. In an alternative embodiment the cell is a permissive eukaryotic cell, preferably a cell derived from fish, preferably from carp, more preferred from *Cyprinus carpio carpio* or *Cyprinus carpio koi.* The cell, which may be either prokaryotic or eukaryotic, may be obtained by infection or transfection of the above mentioned recombinant koi herpesvirus or the above mentioned DNA sequence or the above mentioned vector according to the present invention. The cell may be designated as "transformant". An *E. coli* strain comprising the recombinant koi herpesvirus having a BAC vector sequence incorporated in the viral thymidine kinase gene was deposited at the Belgian Coordinated Collection of Microorganisms (BCCM) (BCCM/LMPB Plasmid Collection, Department of Molecular Biology, Ghent University, Technologiepark 927, 9052 Gent - Zwijnaarde, Belgium). The cell was deposited on July 13, 2007 and was assigned with the deposition number LMBP 5658. This cell is particularly preferred.

The present invention also provides a virus or viral particles produced by the cell according to the present invention.

The present invention further provides a method for the production of infectious particles of recombinant koi herpesvirus (KHV) or Cyprinid herpesvirus 3 (CyHV-3), comprising the step of introduction of any one of the following recombinant koi herpesvirus (KHV) genome or recombinant Cyprinid herpesvirus 3 (CyHV-3) genome into permissive eukaryotic cells: (a) a recombinant koi herpesvirus genome (KHV) or recombinant Cyprinid herpesvirus 3 (CyHV-3) genome comprising a bacterial artificial chromosome (BAC) vector sequence; (b) a recombinant koi herpesvirus genome (KHV) or recombinant Cyprinid herpesvirus 3 (CyHV-3) genome wherein the bacterial artificial chromosome (BAC) vector sequence is excised from the construct of (a); and culturing the host cell to produce recombinant koi herpesvirus (KHV) or recombinant Cyprinid herpesvirus 3 (CyHV-3).

Further, the present invention is also directed to infectious particles generated by the above mentioned method. Further preferred the infectious particles provided are comprising any one of the following: (a) a recombinant koi herpesvirus (KHV) genome or recombinant Cyprinid herpesvirus 3 (CyHV-3) genome comprising a bacterial artificial chromosome (BAC) vector sequence; (b) a recombinant koi herpesvirus (KHV) genome or recombinant Cyprinid herpesvirus 3 (CyHV-3) genome wherein the bacterial artificial chromosome (BAC) vector sequence is excised from the construct of (a).

The above mentioned recombinant koi herpesvirus, the above mentioned DNA sequence the above mentioned vector or the above mentioned infectious particles can be used for the immunization of *Cyprinus carpio carpio* or *Cyprinus carpio koi* individuals by injection or balneation or *per os.* Therefore, the present invention also provides a *Cyprinus carpio carpio* or *Cyprinus carpio koi* individual immunized with the above mentioned recombinant koi herpesvirus or with the above mentioned DNA sequence or with the above mentioned vector or with the above mentioned infectious particles according to the present invention.

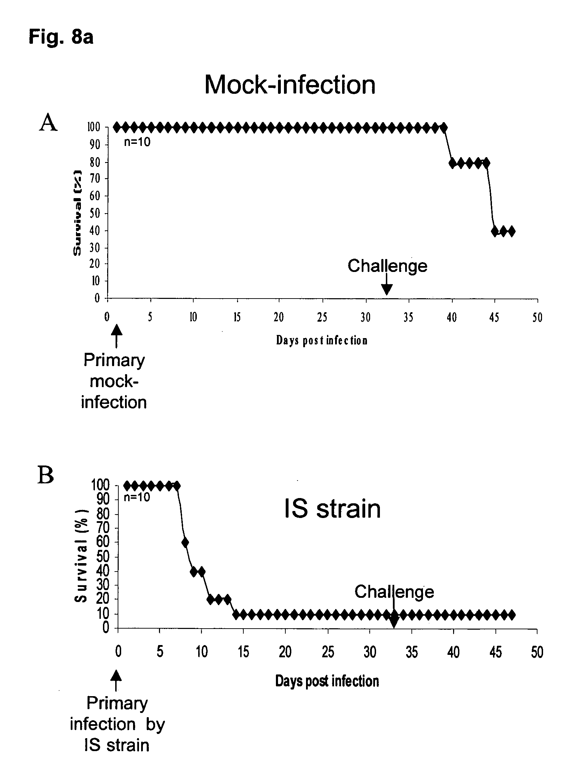

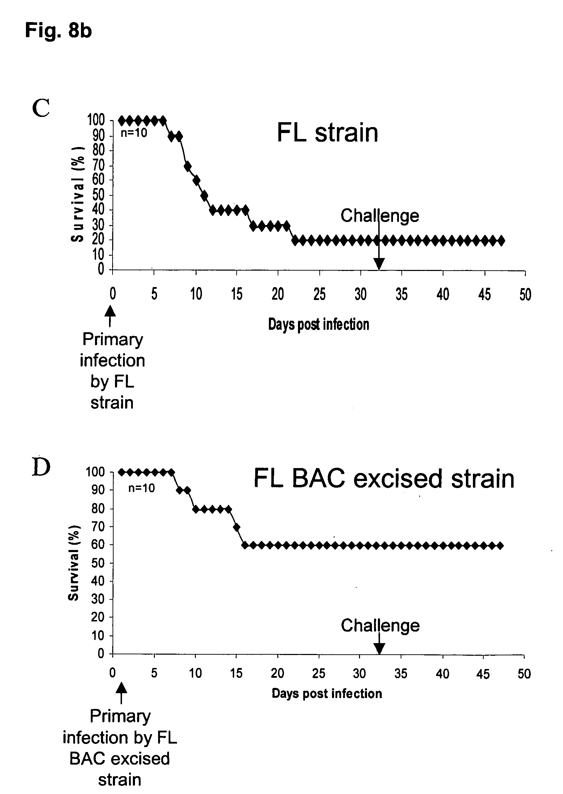

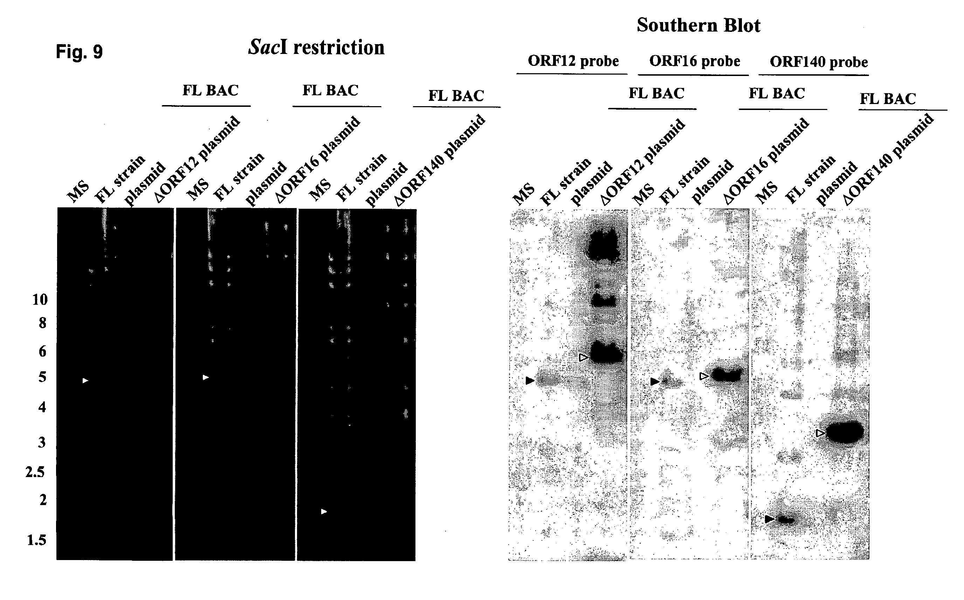

The present invention further provides a koi herpesvirus (KHV) or CyHV-3. This koi herpesvirus isolate, herein also designated as FL strain, was deposited at the Belgian Coordinated Collection of Microorganisms (BCCM) (BCCM/LMPB Plasmid Collection, Department of Molecular Biology, Ghent University, Technologiepark 927, 9052 Gent - Zwijnaarde, Belgium). The FL isolate was deposited on July 13, 2007 and was assigned with the deposition number LMBP 6581 CB. This isolate (FL) shows three important properties: (i) it has very efficient growth properties in cell culture; (ii) it has a lower pathogenicity than comparable wild-type strains; and (iii) it shows a 2 weeks delay of the onset of the disease in 2 g common carps. In order to obtain such an isolate, different KHV field isolates were compared for their growth properties *in vitro* (Fig. 8) and their virulence in koi (7 g) and common (2 g) carps (Fig. 9). Based on the results of these tests, a strain called hereafter FL was selected. Compared to a KHV/CyHV-3 reference strain (IS strain), the FL strain replicated more efficiently *in vitro* and was less pathogenic *in vivo.* When assayed on 7 g koi carps, the IS and the FL strain led to 90% and 80% mortality, respectively (Fig. 9). When assayed on 2 g common carps, the onset of the disease induced by the FL strain was delayed by two weeks in comparison to the IS strain. It should be noted that due to their low age, the 2 g common carps have no adaptive immune system. 7 g common carps already have an adaptive immune response. In view of the observed delay by two weeks compared to the higher virulent KHV IS strain and considering the fact that in common carps the onset of the disease caused by the higher virulent KHV IS strain occurs after about 1 week, the FL strain as such is considered to represent a suitable vaccine for 7 g (and older) common carps.

The KHV FL strain provided in the present invention (deposition number LMBP 6581 CB) may be very useful for the production of an inactivated vaccine and therefore will have a significant industrial potential.

The FL isolate was used to construct the above mentioned recombinant koi herpesvirus according to the present invention. For example, a BAC vector sequence was introduced into the viral thymidine kinase gene, in order to obtain a recombinant koi herpesvirus according to the invention. However, any other KHV isolate can be used in order to construct a recombinant koi herpesvirus according to the present invention. A FL BAC excised strain was assayed as a potential parental vaccine strain on koi carps (see Figure 9). 7 g koi carps were inoculated with the IS (panel (b)), the FL (panel (c)) and the FL BAC excised (panel (d)) strain. Inoculation with the IS, the FL and the FL BAC excised strain led to 90%, 80% and 40% mortality, respectively. This experiment demonstrated that the disruption of the TK locus contribute to a drastic attenuation of the virus. All fishes that survived to the inoculation of the FL BAC excised strain developed a protective immune response against a further challenge with the highly virulent IS strain (see arrow "challenge" in Figure 9). Altogether these data support the potential of the FL BAC clone as a parental plasmid for the development of multi-attenuated recombinant vaccines.

In a further preferred embodiment of the present invention the koi herpesvirus (KHV) or Cyprinid herpesvirus 3 (CyHV-3) deposition number LMBP 6581 CB has the capability to confer immunity on fish, preferably on carps, more preferably on *Cyprinus carpio,* even further preferred on *Cyprinus carpio carpio* and/or *Cyprinus carpio koi,* against a disease caused by koi herpesvirus (KHV) or Cyprinid herpesvirus 3 (CyHV-3).

Preferably, a derivative of the koi herpesvirus (KHV) deposition number LMBP 6581 CB is deficient in one or more viral genes which is/are not essential for replication. More preferred a derivative of the koi herpesvirus (KHV) deposition number LMBP 6581 CB is deficient in at least one gene which contributes to virulence. It is particularly preferred that the derivative of the koi herpesvirus (KHV) deposition number LMBP 6581CB according to the present invention is deficient in at least one gene which contributes to virulence selected from the group consisting of thymidine kinase gene; ORF12: putative tumor necrosis factor (TNF) receptor gene; ORF 16: putative G-protein coupled receptor (GPCR) gene; ORF 134: putative Interleukin 10 homologue gene; ORF140: putative thymidylate kinase gene, or any combination thereof. It is further preferred that the derivative of the koi herpesvirus (KHV) deposition number LMBP 6581 CB according to the present invention is deficient in viral thymidine kinase gene and at least one further gene which contributes to virulence selected from the group consisting of ORF12: putative tumor necrosis factor (TNF) receptor gene; ORF 16: putative G-protein coupled receptor (GPCR) gene; ORF 134: putative Interleukin 10 homologue gene; ORF140: putative thymidylate kinase gene, or any combination thereof. It is further particularly preferred that the derivative of the koi herpesvirus (KHV) deposition number LMBP 6581CB is deficient in all of above mentioned genes. Moreover, the derivative of the koi herpesvirus (KHV) deposition number LMBP 6581 CB may be deficient in further viral genes.

In a further preferred embodiment of the present invention the derivative of the koi herpesvirus (KHV) deposition number LMBP 6581CB is in a live form. Preferably, the derivative of the koi herpesvirus (KHV) deposition number LMBP 6581 CB has the capability to reconstitute infectious particles when introduced into permissive eukaryotic cells or fish individuals preferably in carps, more preferably in *Cyprinus carpio,* even further preferred in *Cyprinus carpio carpio* and/or *Cyprinus carpio koi.*

Further preferred, the derivative of the koi herpesvirus (KHV) deposition number LMBP 6581 CB is in an attenuated form.

In a further preferred embodiment of the present invention the derivative of the koi herpesvirus (KHV) deposition number LMBP 6581 CB results in a mortality rate of 40 % or less, preferably of 20 % or less, more preferred of 10 % or less, further preferred of 1% or less and most preferred of 0.1% or less in fish, preferably in carps, more preferred *Cyprinus carpio carpio* or *Cyprinus carpio* koi infected with said herpesvirus.

In an alternative embodiment of the present invention the derivative of the koi herpesvirus (KHV) deposition number LMBP 6581 CB is in inactivated and non-replicative form or in deficient attenuated form.

The present invention also provides a vaccine for preventive and/or therapeutic treatment of a disease caused by koi herpesvirus (KHV) or CyHV-3.

Further preferred the vaccine is comprising the above mentioned recombinant koi herpesvirus or the above mentioned DNA sequence or the above mentioned vector or the above mentioned infectious particles or the koi herpesvirus (KHV) or CyHV-3 deposition number LMBP 6581 CB or a derivative thereof according to the present invention.

In a further preferred embodiment the above mentioned vaccine is used for preventive and/or therapeutic treatment of a disease caused by koi herpesvirus (KHV) or CyHV-3.

The present invention also provides the use of the above mentioned recombinant koi herpesvirus or of the above mentioned DNA sequence or of the above mentioned vector or of the above mentioned infectious particles or of the above mentioned koi herpesvirus (KHV) or CyHV-3 deposition number LMBP 6581 CB or a derivative thereof for the manufacture of a medicament/vaccine for the preventive and/or therapeutic treatment of a disease caused by koi herpesvirus (KHV) or Cyprinid herpesvirus 3 (CyHV-3).

The vaccine according to the present invention is useful for preventive and/or therapeutic treatment of a disease caused by koi herpesvirus (KHV) or CyHV-3. Any type of vaccine may be produced e.g. inactivated vaccine, inactivated and non-replicative vaccine, recombinant attenuated vaccine, recombinant attenuated vaccine expressing a foreign transgene, DNA vaccine etc. The preferred routes of administration of said vaccine are *per os,* balneation and injection but other routes are not excluded.

Typically, vaccines are prepared as liquid solutions, emulsions or suspensions for injection or delivery in water. For instance, a liquid emulsion or emulsifiable concentrate can be prepared in order to be added to a water tank or bath where the fish are held. Solid (e.g. powder) forms suitable for dissolution in, or suspension in, liquid vehicles or for mixing with solid food, prior to administration may also be prepared. The vaccine may be a lyophilised culture in a ready to use form for reconstitution with a sterile diluent. For instance, lyophilized cells may be reconstituted in 0.9% saline (optionally provided as part of the packaged vaccine product). A preferred formulation of injectable vaccine is an emulsion. Liquid or reconstituted forms of the vaccine may be diluted in a small volume of water (e.g. 1 to 100 volumes) before addition to a pen, tank or bath.

In one preferred embodiment, the vaccine preparation comprising the recombinant KHV or the KHV FL strain is in a dry form, e. g. in a powder form, lyophilized, in a compressed pellet or tablet form, etc. In another embodiment, said virus may be in the form of a tissue culture fluid. Said fluid may be stored at the ambience, preferably at -70°C, most preferably as a solution containing glycerol. In one specific example, the tissue culture fluid contains 20% glycerol.

The recombinant KHV or the KHV FL strain disclosed in the present invention may be converted into a dry form by a number of methods. A particularly preferred form of drying is through lyophilization. Prior to drying, e.g. lyophilization procedure, a variety of ingredients may be added to the medium such as preservatives, anti-oxidants or reducing agents, a variety of excipients, etc. Such excipients may also be added to the dry, e. g. lyophilized active-attenuated virus also after the drying step.

Therefore, in a preferred embodiment, the vaccine according to the invention is in a freeze-dried form. To reconstitute a freeze-dried composition, it is suspended in a physiologically acceptable diluent. Such a diluent can e.g. be as simple as sterile water, or a physiological salt solution. In a more complex form the freeze-dried vaccine may be suspended in an emulsion e.g. as described in EP 1,140,152.

Immunization is typically performed by adding the inactivated virus, inactivated and non-replicative virus, deficient attenuated virus, recombinant attenuated virus, recombinant attenuated virus expressing a foreign transgene, an avirulent virus form such as live-attenuated virus, DNA vaccine to the water containing the fish to be immunized. It is also possible to inject the vaccine preparation directly into the fish. In order to boost the immune response, the preparation may also include a variety of adjuvants, cytokines or other immunostimulants, particularly in the case of preparations that are intended for injection. The vaccine preparation, particularly where it is introduced into the water containing the fish, may also include a variety of stabilizers, antibiotics, etc.

In one embodiment of the invention, the vaccine further comprises an immune modulator adjuvant, stabilizer, antibiotics, immunostimulants or other substances. An adjuvant is an immunostimulatory substance boosting the immune response of the host in a non-specific manner. These adjuvants are known from those already used in human and veterinary medicine. The adjuvant may be hydrophilic adjuvants, e.g. , aluminum hydroxide or aluminum phosphate, or hydrophobic adjuvants, e.g. mineral oil based adjuvants. Adjuvants such as muramyl dipeptides, avridine, aluminium hydroxide, aluminium phosphate, oils, oil emulsions, saponins, dextran sulphate, glucans, cytokines, block co-polymers, immunostimulatory oligonucleotides and others known in the art may be admixed with the inactivated viral supernatant. A preferred adjuvant is Freund's Incomplete Adjuvant (FIA). The amount of adjuvant added depends on the nature of the adjuvant itself. FIA may advantageously be emulsified with inactivated viral supernatant in a ratio of about 1:1 by volume.

Examples of adjuvants frequently used in fish farming are muramyldipeptides, lipopolysaccharides, several glucans and glycans and Carbopol® (a homopolymer). Suitable adjuvants are e.g. water in oil (w/o) emulsions, o/w emulsions and w/o/w double-emulsions. Oil adjuvants suitable for use in w/o emulsions are e.g. mineral oils or metabolisable oils. Mineral oils are e.g. Bayol®, Marcol® and Drakeol®; metabolisable oils are e.g. vegetable oils, such as peanut oil and soybean oil, or animal oils such as the fish oils squalane and squalene. Alternatively a vitamin E (tocopherol) solubilisate as described in EP 382,271 may advantageously be used. Very suitable o/w emulsions are e.g. obtained starting from 5-50 % w/w water phase and 95-50 % w/w oil adjuvant, more preferably 20-50 % w/w water phase and 80-50 % w/w oil adjuvant are used. The amount of adjuvant added depends on the nature of the adjuvant itself, and information with respect to such amounts provided by the manufacturer.

In a preferred embodiment the vaccine according to the invention additionally comprises a stabiliser. A stabilizer can be added to a vaccine according to the invention e.g. to protect it from degradation, to enhance the shelf-life, or to improve freeze-drying efficiency. Useful stabilizers are i.a. SPGA (Bovarnik et al., 1950, J. Bacteriology, vol. 59, p. 509), skimmed milk, gelatine, bovine serum albumin, carbohydrates e.g. sorbitol, mannitol, trehalose, starch, sucrose, dextran or glucose, lactoses, proteins such as albumin or casein or degradation products thereof, and buffers, such as alkali metal phosphates.

Antibiotics such as neomycin and streptomycin may be added to prevent the potential growth of germs. The immunostimulants may enhance protection afforded by vaccines and provide non-specific protection against a number of diseases. Many chemicals and other substances have been reported as having immunostimulating properties in fish, i.e., chemicals and glucans, extracts from algae-algines (alginic acid silylic esters) and cytokines.

In addition, the vaccine may comprise one or more suitable surface-active compounds or emulsifiers, e.g. Span ® or Tween®. The vaccine may also comprise a so-called "vehicle". A vehicle is a compound to which the KHV virus (either in form of a virus particle or in form of DNA) according to the invention adheres, without being covalently bound to it. Such vehicles are i.a. bio-microcapsules, micro-alginates, liposomes and macrosols, all known in the art. A special form of such a vehicle is an Iscom, described above. It goes without saying that admixing other stabilizers, carriers, diluents, emulsions, and the like to vaccines according to the invention are also within the scope of the invention. Such additives are for instance described in well-known handbooks such as: "Remington: the science and practice of pharmacy" (2000, Lippincot, USA, ISBN: 683306472), and: "Veterinary vaccinology" (P. Pastoret et al. ed., 1997, Elsevier, Amsterdam, ISBN: 0444819681 ).

The recombinant KHV or the KHV FL strain when used in its dry form in a vaccine may further include a reconstitution fluid, preferably sterile water, saline or physiological solution. It may also contain small amounts of residual materials from the manufacturing process such as cell proteins, DNA, RNA, etc. While these materials are not additives per se, they may nonetheless be present in the vaccine formulation.

The invention further relates to a method for immunizing fish against the viral infection caused by KHV described hereinbefore, said method comprising administration to susceptible fish a vaccine formulation comprising the recombinant KHV or the KHV FL strain according to the present invention, being administered in an amount sufficient to induce immunity to subsequent infection by KHV.

The vaccine may be administered to the aquaculture fish individually-orally, e.g. through their feed or by forced oral administration, or by injection (intramuscular or the intraperitoneal route). Alternatively the vaccine may be administered simultaneously to the entire fish population contained in a body of water by spraying, dissolving and/or immersing the vaccine. These methods are useful for vaccination of all kinds of fish, e.g., food and ornamental, and in various environments such as, and not limited to, ponds, aquariums, natural habitat and fresh water reservoirs.

A further aspect of the invention relates to a DNA vaccine comprising the recombinant KHV according to the invention. Nucleic acid vaccines (or gene-or genetic-vaccines as they are called) may require a targeting- or a delivery vehicle to target or protect it, or to assist in its uptake by (the cells of) the host. Such vehicles may be biologic or synthetic, and are for instance bacteriophages, virus-like particles, liposomes, or micro-, powder-, or nano particles.

DNA vaccines can easily be administered through intradermal application e.g. using a needle-less injector such as a GeneGun®. This way of administration delivers the DNA directly into the cells of the animal to be vaccinated. A preferred amount of a nucleic acid, a DNA fragment, or a recombinant DNA molecule according to the invention, comprised in a pharmaceutical composition according to the invention (as outlined below) is in the range between 10 pg and 1000 µg. Preferably, amounts in the range between 0.1 and 100 µg are used. Alternatively, fish can be immersed in solutions comprising e.g. between 10 pg and 1000 µg/ml of the DNA to be administered. All these are well-known in the art.

Similarly, a targeting- or delivery vehicle comprising a nucleic acid, a DNA fragment, or a recombinant DNA molecule according to the invention, is within the scope of a carrier according to the invention. These uses will result in nucleic acid being delivered, or protein being expressed inside the target organism or its cells.

In a further aspect, the invention relates to a method of vaccination of fish by administering to such organism a vaccine according to the invention, in a pharmaceutically effective amount and in a pharmaceutically acceptable carrier. A "pharmaceutically effective amount" is described in detail below.

A vaccine according to the invention may take any form that is suitable for administration in the context of aqua-culture farming, and that matches the desired route of application and desired effect. Preparation of a vaccine according to the invention is carried out by means conventional for the skilled person.

Preferably the vaccine according to the invention is formulated in a form suitable for injection or for immersion vaccination, such as a suspension, solution, dispersion, emulsion, and the like. Commonly such vaccines are prepared sterile.

The dosing scheme of the application of a vaccine according to the invention to the target organism can be in single or multiple doses, which may be given at the same time or sequentially, in a manner compatible with the dosage and formulation and in such an amount as will be immunologically effective. It is well within the capacity of the skilled person to determine whether a treatment is "immunologically effective", for instance by administering an experimental challenge infection to vaccinated animals, and next determining a target animals' clinical signs of disease, serological parameters, or by measuring reisolation of the pathogen.

What constitutes a "pharmaceutically effective amount" for a vaccine according to the invention that is based upon a nucleic acid according to the invention is dependent on the desired effect and on the target organism. Determination of the effective amount is well within the skills of the routine practitioner. A preferred amount of a nucleic acid, a DNA fragment, or a recombinant DNA molecule according to the invention, comprised in a pharmaceutical composition according to the invention, has been described above.

A preferred amount of a vaccine comprising recombinant KHV virus or KHV FL strain according to the invention is expressed for instance as plaque forming units (pfu), colony forming units (cfu) or tissue culture infective dose 50% (TCID50). For instance for a live viral vector a dose range between 1 and 10¹⁰ plaque forming units (pfu) per animal dose may advantageously be used; preferably a range between 10² and 10⁶ pfu/dose. A suitable dosage range of virus is from about 10² to 10⁹ TCID50 (tissue culture infectious dose affecting 50% of cultures inoculated) per unit dose, preferably about 10⁴ to 10⁸ TCID50 per unit dose, more preferably about 10⁶ to 10⁷ TCID50 per unit dose, most preferably about 10⁷ TCID50 per unit dose. Preferably a single dosage unit is administered to the fish to be treated. Many ways of administration can be applied, all known in the art. The vaccines according to the invention are preferably administered to the fish via injection (intramuscular or the intraperitoneal route), immersion, dipping or per os. The protocol for the administration can be optimized in accordance with standard vaccination practice.

Preferably the vaccine is administered via immersion or per os. This is especially efficient in case of the use of such vaccines in the setting of commercial aqua-culture farming.

The age and the weight, of the fish to be vaccinated is critical. Although in principle it is evidently favorable to vaccinate against KHV as early as possible to prevent a field infection, carps having a weight of less than 7 g however are assumed to be insufficiently immune competent, meaning that they do not show adaptive immune response. Therefore, in practice, one would try to vaccinate fish having a weight of 7 g and more.

The vaccines according to the invention, described above, contribute to active vaccination, i.e. they trigger the host defense system.

The present invention further provides a method for the preventive and/or therapeutic treatment of a disease caused by koi herpesvirus (KHV) or CyHV-3 comprising the introduction of an therapeutic effective amount of the above mentioned recombinant koi herpesvirus or of the above mentioned DNA sequence or of the above mentioned vector or of the above mentioned infectious particles or of the above mentioned koi herpesvirus (KHV) or CyHV-3 deposition number LMBP 6581 CB or a derivative thereof into fish, preferably carps, more preferably *Cyprinus carpio carpio* or *Cyprinus carpio koi* individuals for the preventive and/or therapeutic treatment of a disease caused by koi herpesvirus (KHV) or CyHV-3.

### Modes for carrying out the invention

The present invention provides a recombinant koi herpesvirus (KHV) or Cyprinid herpesvirus 3 (CyHV-3), which preferably is immunogenic in fish, preferably in carps, more preferably in *Cyprinus carpio.* In one embodiment of the invention the recombinant koi herpesvirus has the capability to reconstitute infectious particles when introduced into permissive eukaryotic cells. The inventors of the present invention achieved the cloning of the KHV genome as a stable and infectious BAC clone. This goal was achieved by insertion of a modified *loxP-*flanked BAC cassette into the thymidine kinase locus. This insertion allowed the production of a KHV BAC clone stably maintained in bacteria and able to regenerate virions when transfected into permissive cells.

Further, the *loxP*-flanked BAC cassette can be excised from the genome of reconstituted virions by growing them on permissive cells expressing Cre recombinase. The present invention therefore is the first to provide the BAC cloning of KHV genome and is the first to provide the BAC cloning of a herpesvirus genome as large as KHV. The availability of the KHV BAC is an important advance that will allow the identification of viral genes involved in KHV pathogenesis, as well as the production of attenuated recombinant candidate vaccines.

The present invention was achieved by cloning of the KHV genome by the insertion of a modified *loxP*-flanked BAC cassette into the thymidine kinase (TK) locus which was postulated to be non-essential for KHV replication. This insertion led to a BAC recombinant virus whose genome was stably maintained in bacteria and was able to regenerate virions when transfected into permissive cells.

A central advantage of the BAC cloning system over cosmid vectors used in prior art is that the F-plasmid is present in only one or a maximum of two copies per cell reducing the potential for recombination between DNA fragments and, more importantly, avoiding the lethal overexpression of cloned bacterial genes. However, the presence of the BAC as just a single copy means that plasmid DNA has to be extracted from a large volume of culture to obtain sufficient DNA for sequencing and it is described here in the examples as simplified protocol to achieve this.

The term "BAC vector" refers to a plasmid which is produced using F plasmid of *E. coli* and a vector which can stably maintain and grow a large size DNA fragment of about 300 kb or more in bacteria, such as *E. coli* and the like. The BAC vector contains at least a region essential for the replication of the BAC vector. Examples of such a region essential for replication include, but are not limited to, the origin of replication of F plasmid and variants thereof.

As used herein, the term "BAC vector sequence" refers to a sequence comprising a sequence essential for the function of a BAC vector. Optionally, the BAC vector sequence may further comprise a "recombination protein-dependent recombinant sequence" and/or a "selectable marker".

As used herein, the term "homologous recombination" indicates that two different homologous nucleic acid molecules encounter each other, crossover occurs, and a new combination of nucleic acid is generated. As used herein, the term "sequence mediating homologous recombination" refers to a sequence which causes homologous recombination which is dependent from a specific recombination protein, which is catalyzing, carrying out or assisting in homologous recombination. Such a recombination protein preferably acts specifically on a "sequence mediating homologous recombination" and does not act on other sequences.

Examples of representative pairs of such a sequence and recombination protein catalyzing, carrying out or assisting homologous recombination include, but are not limited to: a combination of a bacteriophage P1-derived *loxP* (locus of crossover of P1) sequence and a Cre (cyclization recombination) protein, a combination of Flp protein and FRT site, a combination of C31 and attB or attP, a combination of resolvase and res site.

As used herein, the term "selectable marker" refers to a gene which functions as an index for selection of a host cell containing a BAC vector. Examples of a selectable marker include, but are not limited to, fluorescent markers, luminiscent markers, and drug selectable markers. An example of a "fluorescent marker" is, but is not limited to, a gene encoding a fluorescent protein, such as a green fluorescent protein (GFP). An example of a "luminiscent marker" is, but is not limited to, a gene encoding a luminescent protein, such as luciferase. An example of a "drug selectable marker" is, but is not limited to, a gene encoding a protein selected from the group consisting of: dihydrofolate reductase gene, glutamine synthase gene, aspartic acid transaminase, metallothionein (MT), adenosine deaminase (ADA), adenosine deaminase (AMPD1, 2), xanthine-guanine-phosphoribosyl transferase, UMP synthase, P-glycoprotein, asparagine synthase, and ornithine decarboxylase. Examples of a combination of a drug selectable marker and a drug include: a combination of dihydrofolate reductase gene (DHFR) and methotrexate (MTX), a combination of glutamine synthase (GS) gene and methionine sulfoximine (Msx), a combination of aspartic acid transaminase (AST) gene and N-phosphonacetyl-L-aspartate) (PALA), a combination of MT gene and cadmium (Cd²⁺), a combination of adenosine deaminase (ADA) gene and adenosine, alanosine, or 2'-deoxycoformycin, a combination of adenosine deaminase (AMPD1, 2) gene and adenine, azaserine, or coformycin, a combination of xanthine-guanine-phosphoribosyltransferase gene and mycophenolic acid, a combination of UMP synthase gene and 6-azaulysine or pyrazofuran, a combination of P-glycoprotein (P-gp, MDR) gene and multiple drugs, a combination of asparagine synthase (AS) gene and -aspartyl hydroxamic acid or albizziin, and a combination of ornithine decarboxylase (ODC) gene and - difluoromethyl-ornithine (DFMO).

Conveniently, the BAC cassette as used herein further contains a gene which confers resistance to chloramphenicol (CmR), a selection marker in prokaryotic cells. Further comprised in the BAC cassette is a resistance gene for G418 (NeoR) which is in fusion with EGFP (EGFP-Neo). As result, this fusion protein between EGFP and Neo confers EGFP fluorescence and resistance to G418. The EGFP-Neo gene is under the control of the HCMV IE promoter and represents a selection marker in eukaryotic cells.

As used herein, the term "expression vector" refers to a nucleic acid sequence comprising a structural gene and a promoter for regulating expression thereof, and in addition, various regulatory elements in a state that allows them to operate within host cells. The regulatory element may include, preferably, terminators, selectable markers such as drug-resistance genes (e.g., a kanamycin resistance gene, a hygromycin resistance gene, etc.), and enhancers. It is well known to those skilled in the art that the type of an organism, expression vector and the type of a regulatory element may vary depending on the host cell.

As used herein, the term "vector" refers to a vector which can transfer a polynucleotide sequence of interest to a target cell. Examples of such a vector include vectors which are capable of self replication or capable of being incorporated into a chromosome within host cells (e.g., prokaryotic cells, yeast, animal cells, plant cells, insect cells, whole animals, and whole plants), and contain a promoter at a site suitable for transcription of a polynucleotide of the present invention.

As used herein, the term "promoter" refers to a base sequence which determines the initiation site of transcription of a gene and is a DNA region which directly regulates the frequency of transcription. Transcription is started by RNA polymerase binding to a promoter.

As used herein, the terms "transformation", "transduction" and "transfection" are used interchangeably unless otherwise mentioned, and refers to introduction of a nucleic acid into host cells. As a transformation method, any technique for introducing DNA into host cells can be used, including various well-known techniques, such as, for example, the electroporation method, the particle gun method (gene gun), the calcium phosphate method, and the like.

As used herein, the term "transformant" refers to the whole or a part of an organism, such as a cell, which is produced by transformation. Examples of a transformant include prokaryotic cells, yeast, animal cells, plant cells, insect cells and the like. Transformants may be referred to as transformed cells, transformed tissue, transformed hosts, or the like, depending on the subject. As used herein, all of the forms are encompassed, however, a particular form may be specified in a particular context.

### Description of the figures

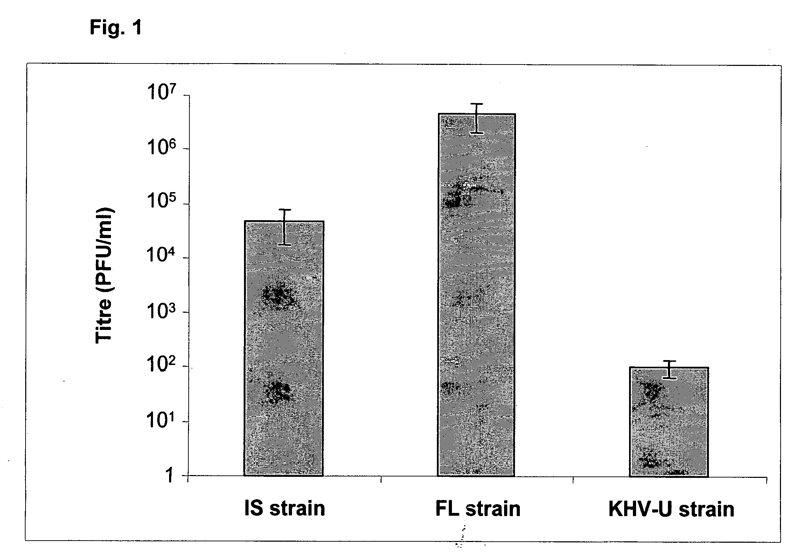

**Figure 1** shows the *in vitro* comparison of the growth properties of three KHV field isolates. CCB cells were infected with the indicated KHV field isolate. Five days post infection, supernatants of infected cultures were harvested and the amount of infectious virus was determined by plaque assay on CCB.

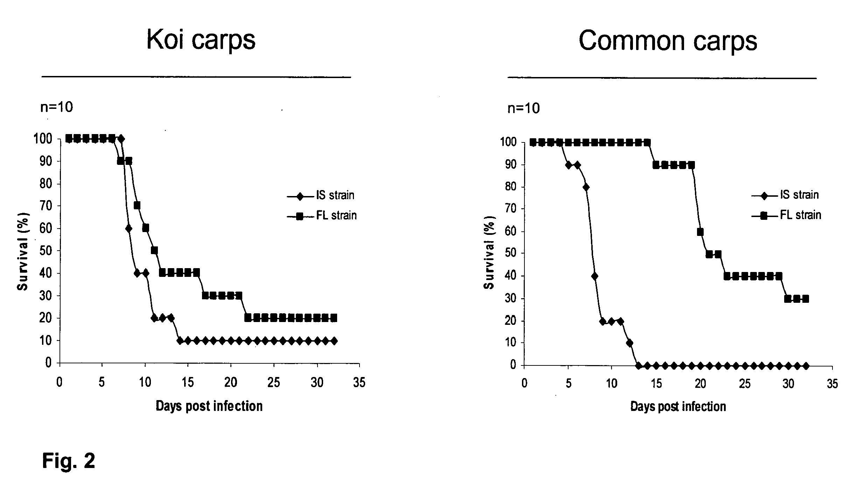

**Figure 2** shows the comparison of the pathogenicity of the KHV IS strain versus the KHV FL strain. On day 0, 7 g koi carps (n=10) and 2 g common carps (n=10) were infected by intraperitoneal injection with either the IS strain (◆) or the FL strain (■). Percentage of survivors is expressed according to days post infection.

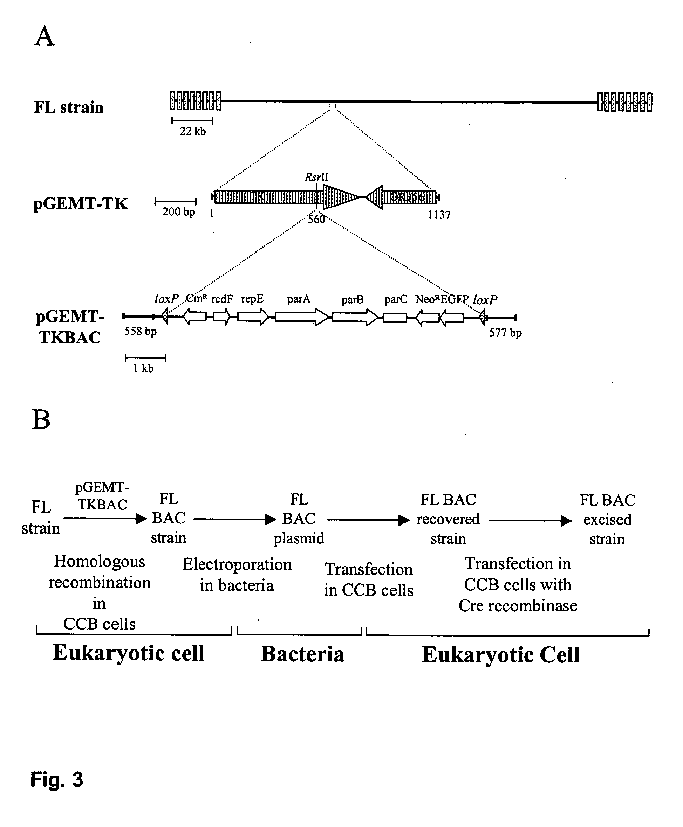

**Figure 3** is a schematic representation of the strategies used to produce infectious KHV FL BAC plasmid. (A) The genome of the KHV FL strain, flanked by two direct terminal repeats is shown at the top. A *loxP*-flanked BAC cassette was inserted into the *Rsr*II sites of TK (thymidine kinase) ORF of the pGEMT-TK vector resulting in pGEMT-TKBAC. In the latter vector, the BAC cassette is flanked by KHV homologous region of 558 bp and 577 bp. (CmR: confers resistance to chloramphenicol. This gene is under the control of a prokaryotic promotor. RedF: site-specific recombinase gene (it is a truncated version and whether the product of this gene is functional is not known); repE: mediates assembly of a replication complex at Ori2; parA, parB, parC: these three elements insure that the BAC remains as a single copy per bacterial cell; NeoR: resistance gene for G418 (fusion with EGFP); EGFP-Neo: This open reading frame encodes a fusion protein between EGFP and Neo. The expression product of this ORF confers EGFP fluorescence and resistance to G418. This ORF is under the control of the HCMV IE promotor. This gene is a selection marker in eukaryotic cells. Ori2: origin of replication of *E.coli* factor F. **(B)** Flowchart of stages performed to produce KHV FL BAC plasmid, to control its infectivity and to demonstrate the possibility of removing the *loxP*-flanked BAC cassette from the genome of reconstituted virus.

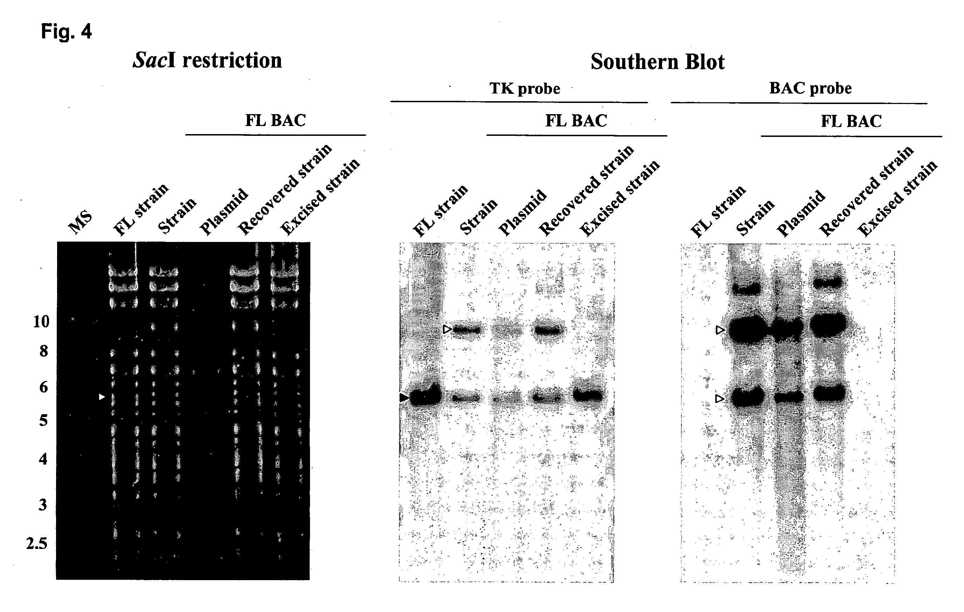

**Figure 4** shows a structural analysis of KHV FL BAC plasmid and derived strains. The KHV FL BAC plasmid and the genome of the KHV FL, FL BAC, FL BAC recovered and FL BAC excised strains were analyzed by *Sac*l restriction (left panel) and further tested by Southern blot using probes corresponding to the TK ORF (middle panel) or the BAC cassette (right panel). Arrows and open arrows indicate restriction fragments containing the TK ORF and the BAC cassette, respectively. Markers sizes (MS) in kb are indicated on the left.

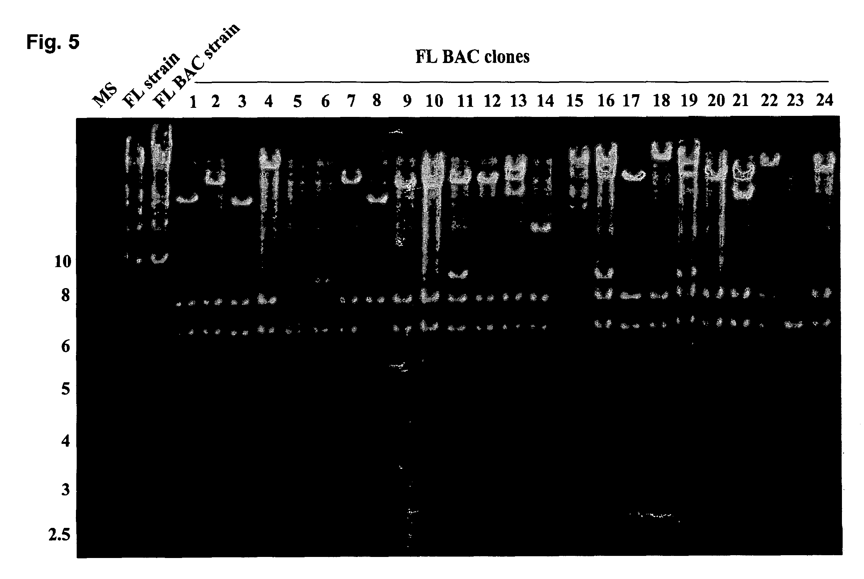

**Figure 5** shows the heterogeneity among KHV FL BACFL plasmids. BAC plasmids were isolated from twenty-four independent *E. coli* clones and analyzed by *Hind*III. KHV parental (FL strain) and FL BAC strains were used as controls. Markers sizes (MS) in kb are indicated on the left.

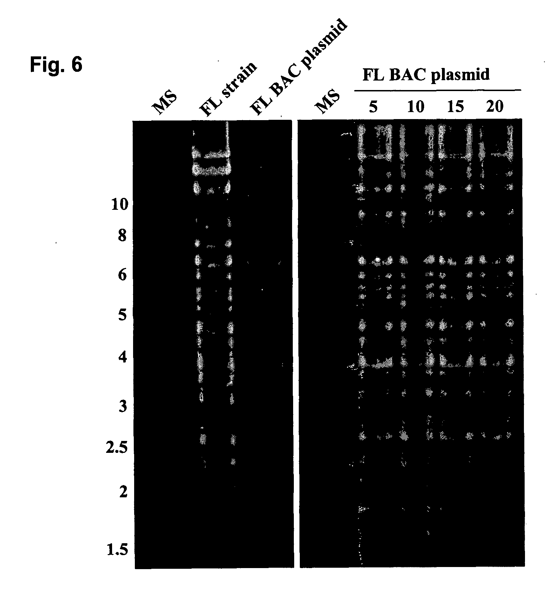

**Figure 6** shows the experiments on the stability of the KHV FL BAC plasmid in *E. coli.* DH10B cells containing KHV FL BAC plasmid were serially cultured for 20 consecutive days. At the indicated days, BAC DNAs were prepared as described in materials and methods then digested by *Sac*I. The KHV parental strain (FL strain) was used as control. Markers sizes (MS) in kb are indicated on the left.

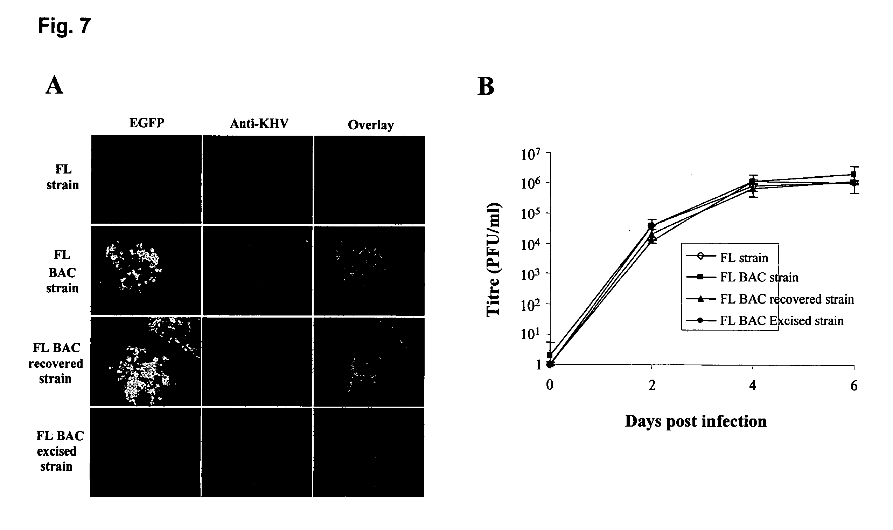

**Figure 7** shows the characterization of KHV derived strains. (A) Epifluorescent analysis of viral syncitia. CCB cells were infected (MOI of 0.1 PFU/cell) with KHV FL (i-iii), FL BAC (iv-iv), FL BAC recovered (vii-ix) and FL BAC excised (x-xii) strains and overlaid with DMEM containing 10 % FCS and 0.6 % (w/v) carboxymethylcellulose (Sigma) to obtain isolated syncitia. Seven days pi, syncitia were revealed by indirect immunofluorescent staining using anti-KHV polyserum and GAR 568 as the primary and secondary antibodies, respectively. The sets of three horizontal panels (i-iii, iv-vi, vii-ix and x-xii) represent analyses of the same syncitium. Panels (i), (iv), (vii), (x) and panels (ii), (v), (viii), (xi) were analyzed for EGFP and GAR 568 fluorescent emissions, respectively. The merged EGFP and Alexa signals are shown in panels (iii), (vi), (ix) and (xii). The side of each panel corresponds to 200 µm. **(B)** Replication kinetics of KHV recombinant strains were compared with those of the parental KHV FL strain as described in materials and methods. The data presented are the means of triplicate measurements.

**Figure 8** shows the attenuation of the KHV FL BAC excised strain *in vivo.* On day 0, four groups, each consisting of 10 koi carps (7 g), were infected by intraperitoneal injection with **(B)** the IS strain **(C)** the FL strain and **(D)** the FL BAC excised strain. Koi injected with mock-infected culture medium were used as control **(A)**. On day 32 post infection, survival fish were challenged by intraperitoneal injection with the IS strain (see arrow "challenge"). The percentage of survivors is expressed according to days post infection.

**Figure 9** shows the structural analysis of KHV FL BAC *gal*K recombinant plasmids. The KHV FL BAC plasmid and derived *ga*/K recombinant plasmids were analyzed by *Sac*I restriction (left panel) and further tested by Southern blot (right panel) using probes corresponding to ORF12 (putative tumor necrosis factor (TNF) receptor gene), ORF16 (putative G-protein coupled receptor (GPCR) gene) or ORF140 (putative thymidylate kinase gene.). KHV WT (FL strain) was used as control. Arrows and open arrows indicate restriction fragments containing the corresponding ORF and the *gal*K cassette, respectively. Markers sizes (MS) in kb are indicated on the left.

### Examples

**Materials and Methods**

**a) Cells and viruses**. Cyprinus carp brain cells (CCB) (Neukirch et al., Bull. Eur. Ass. Fish. Pathol., 19 (5), 221-224 (1999)) were cultured in minimum essential medium (MEM, Invitrogen) containing 4.5 g/I glucose (D-glucose monohydrate, Merck) and 10 % fetal calf serum (FCS). Cells were cultured at 25°C in a humid atmosphere containing 5 % CO₂. The KHV FL strain was isolated from the kidney of a fish which died from KHV (Belgium). KHV IS and KHV-U strains were kindly provided by M. Kotler (Hebrew University-Hadassah Medical School, Jerusalem, Israel) and R. Hedrick (University of California, School of Veterinary Medicine, USA) respectively. (Ronen et al., Vaccine, 21 (32), 4677-4684 (2003); Hedrick et al., Bull. Fish. Res. Agen., S2, 1-7 (2005))

**b) Growth assay.** Triplicate cultures of CCB cells were infected with KHV FL, KHV IS and KHV-U strains (Multiplicity of infection of 0.5 PFU/cell). After an incubation period of 2 h, cells were washed with PBS and then overlaid with Dulbecco's modified essential medium (DMEM, Invitrogen) containing 4.5 g/I glucose and 10 % FCS. Five days post infection (pi), supernatants of infected cultures were harvested and the amount of infectious virus was determined by plaque assay on CCB cells as described previously (Gillet et al., J Gen Virol, 86, 907-917 (2005)).

**c) BAC cloning of KHV.** Firstly, a 1137 bp DNA fragment, corresponding to TK open reading frame (ORF) and ORF56 of the KHV genome, was amplified by PCR using KHV FL DNA as template. The following primers were used for the amplification: the forward primer 5'-ATGGCTATGCTGGAACTGGTG-3' and the reverse primer 5'-CTCAACAGGGAAGAGTGGCG-3' corresponding to nucleotides 1-21 of KHV TK ORF and nucleotides 279-297 of ORF56 respectively (Genbank accession no. DQ177346). The amplification product was TA cloned into the pGEMT-easy vector (promega) resulting in pGEMT-TK (Fig. 1A). Three independent clones were sequenced. A BAC cassette was released by *Pmel* digestion of a pBeloBACModified-EGFPNeo vector (Dewals et al., J Gen Virol, 87, 509-517 (2006)) then ligated into the *Rsr*II site of the pGEMT-TK vector resulting in pGEMT-TKBAC vector (**Fig. 1A**) in which the BAC cassette was flanked by sequences homologous to KHV genome. These homologous regions of 558 bp and 577 bp were used to produce a KHV FL BAC recombinant strain by homologous recombination in eukaryotic cells (**Fig. 1B**). Briefly, freshly seeded CCB cells were infected with KHV at a multiplicity of infection (MOI) of 0.5 plaque forming units (PFU)/cell. After an incubation period of 2 h, cells were transfected with the circular pGEMT-TKBAC vector using Lipofectamine Plus (Invitrogen). Four days post-infection (pi), to select BAC-recombinant virus, supernatant of infected cells was inoculated on fresh uninfected cells (10⁶ cells per 9.5 cm²) in the presence of G418 (final concentration 500 µg/ml). This step was repeated three times until a pure BAC-recombinant population was obtained. Circularized form of the viral BAC-recombinant genome was extracted 20 h pi as described previously (Morgan et al., Avian Dis, 34, 345-351. (1990)) and 2 µg DNA was electroporated (2250 V, 132 Ω, 40 µF) into *E. coli* DH10B cells (Invitrogen) as described elsewhere. Electroporated cells were plated on solid LB plate with chloramphenicol (17 µg/ml).

**d) Excision of the BAC cassette from the KHV BAC genome.** The pEFIN3-NLS-Cre plasmid (Gillet et al., J Gen Virol, 86, 907-917 (2005)) was co-transfected into CCB cells together with the DNA of KHV FL BAC recovered strain. Seven days post-transfection, non-EGFP-expressing viral plaques were picked and cloned by four successive rounds of plaque purification.

**e) Southern blotting.** Southern blot analysis was performed as described previously (Markine-Goriaynoff et al., J Gen Virol, 85, 355-367 (2004)) using pGEMT-TK and pBeloBACModified-EGFPNeo vectors as probes.

**f) Indirect immunofluorescence staining.** CCB cells were fixed and permeabilized with acetone-ethanol (50:50) for 10 min at -20 °C. Immunofluorescent staining (incubation and washes) was performed in PBS containing 10 % FCS. Samples were incubated at 25°C for 45 min with a rabbit anti-KHV polyserum (diluted 1:10,000). After three washes, samples were incubated at 25°C for 30 min with Alexa Fluor 568 goat anti-rabbit IgG (H+L) (GAR 568, diluted 1:1000, Molecular Probes) as the secondary conjugate.

**g) Microscopy analysis.** Epifluorescent microscopy analysis was performed with a DMIRBE microscope (Leica) equipped with a DC 300F CCD camera (Leica) as described previously.

**h) Multi-step growth curves.** Triplicate cultures of CCB cells were infected at a MOI of 0.5 PFU/cell. After an incubation period of 2 h, cells were washed with PBS and then overlaid with Dulbecco's modified essential medium (DMEM, Invitrogen) containing 4.5 g/I glucose and 10 % FCS. Supernatant of infected cultures was harvested at successive intervals after infection and the amount of infectious virus was determined by plaque assay on CCB cells as described previously (Gillet et al., J Gen Virol, 86, 907-917 (2005)).

**i) Induction of KHV disease in fish.** Specific-pathogen-free koi and common carps, with an average weight of 7 g and 2 g respectively, were kept in 60-liter tanks with a water temperature of 24°C. To test the pathogenicity of KHV field strains *in vivo,* two groups of fish were used each comprising 10 koi carps and 10 common carps. Koi carps were infected by intraperitoneal (i.p.) injection with 0.1 ml containing 300 PFU of the KHV IS or the KHV FL strain. Common carps were infected by bathing the fish in a 300 ml tank, to which either the KHV IS or the KHV FL strain was added to achieve a final concentration of 30 PFU/ml. After 2 h under these conditions, fishes were transferred into large tanks. To compare the pathogenicity of the KHV FL BAC excised strain to the pathogenicity of the parental KHV FL strain, four groups of fish were used each comprising 10 koi carps. Koi carps were infected by intraperitoneal (i.p.) injection with 0.1 ml containing 300 PFU of the KHV IS strain, the KHV FL strain or the KHV FL BAC excised strain. The control group (mock-infected) was injected with culture medium under the same conditions. Fishes were examined daily for clinical signs of KHV disease. The animal study was accredited by the local ethics committee of the University of Liège (Belgium).

**j) Production of KHV FL BAC recombinant plasmids using a *gal*K positive selection in bacteria.** KHV FL BAC recombinant plasmids deleted for either ORF12 (putative tumor necrosis factor (TNF) receptor gene), ORF16 (putative G-protein coupled receptor (GPCR) gene) or ORF140 (putative thymidylate kinase gene) were produced as follows. Firstly, a 1331 bp DNA fragment, corresponding to *gal*K ORF flanqued by 50bp-homologous arms of either ORF12, ORF16 or ORF140 (Genbank accession no. DQ177346) was amplified by PCR using *pgal*K vector as template (Warming et al., Nucleic Acids Research, 33 (4) (2005)). The following primers were used for the amplification: ORF12: forward 5'-ATGAAGCTGCTTGTGATTCTGGGTCTTGGACTCTGCTAC CTG GCTGTTCACCTGTTGACAATTAATCATCGGCA-3' and reverse 5'-TCAAC TTCTCTTTGGTTTTCTGCACATATTCGTCCCCCCCACGGTTTTCATCAGCACT GTCCTGCTCCTT-3' primer; ORF16: forward 5'-ATGAAACCTCTGGGTCTTTTT GTTTCTGTGCTCGGGCTGCTTGCCCTGTCCCTGTTGACAATTAATCATCGGCA -3' and reverse 5'-TCATAGGACGCCATCGGTTGAGTTCGCTGCGGCTGCGA CTCCCAGTCCTCTCAGCACTGTCCTGCTCCTT-3' primer; ORF140: forward 5'-ATGGAACCCGAGACCTCGACAGCGAGAACCGCCGAGCATGCCCTTTCATG CCTGTTGACAATTAATCATCGGCA-3' and reverse 5'-TCAAGACCATAAAGTG GGAATGGTGTCGTCGAAGCCGTCTCTGGAGTCGTTCAGCACTGTCCTGCTCC TT-3' primer. The amplification product was purified (QlAquick Gel Extraction Kit) and stored at -20°C until use. In parallel, 1 µg of KHV FL BAC plasmid was electroporated (2500 V, 100 Ω, 25 µF) into *E. coli* SW102 cells (Warming et al., Nucleic Acids Research, 33 (4), (2005)). Electroporated cells were grown at 32°C in liquid LB medium with chloramphenicol (17 µg/ml). Next, electrocompetent SW102 cells containing the KHV FL BAC plasmid were electroporated with 2.5 ng of the PCR products described above. Electroporated cells were plated on solid M63 minimal medium supplemented with 20 % galactose and chloramphenicol (17 µg/ml) as described elsewhere to select bacteria in which homologous recombination occurred (Warming et al., Nucleic Acids Research, 33 (4), (2005)). Finally, colonies obtained were streaked onto MacConkey indicator plates as described elsewhere to confirm the production of *gal*K positive (Warming et al., Nucleic Acids Research, 33 (4), (2005)).

### Example 1: The KHV FL strain has intrinsic properties as a parental strain for production of derived attenuated recombinant vaccines.

Field strains were tested for their growth properties *in vitro* and their virulence *in vivo.* The KHV FL strain was selected at the end of this screening process as parental strain for production of derived attenuated recombinant strains. Figure 1 illustrates the growth properties of the KHV FL strain as compared to two KHV reference strains (IS and KHV-U strain), the FL strain replicated more efficiently *in vitro.* Figure 2 shows that *in vivo* the KHV FL strain was less pathogenic than the KHV IS strain. Indeed, when assayed on 7 g koi carps, the IS and the FL strain led to 90% and 80% mortality, respectively (Fig. 2). Interestingly, when assayed on 2 g common carps, the onset of the disease induced by the FL strain was delayed by two weeks in comparison to the IS strain. It should be noted that due to their low age the 2 g common carps have no adaptive immune system. 7 g common carps already have an adaptive immune response. In view of the observed delay by two weeks compared to the higher virulent KHV strain and considering the fact that in common carps the onset of the disease caused by the higher virulent KHV strain occurs after about 1 week, the FL strain as such is considered to represent a suitable vaccine for 7 g (and older) common carps. Based on the results of these tests, the FL strain was selected as parental strain for further experiments (FL stands for François Lieffrig who isolated the strain) [deposition number LMPB 6581 CB].

### Example 2: Cloning of the KHV genome in E. coli.

In order to produce KHV recombinant strains, the genome of the KHV FL strain was cloned as a BAC. The approach depicted in **Fig. 3** was used to BAC clone KHV. This approach required as a first step the insertion of the BAC cassette into the KHV genome by homologous recombination in eukaryotic cells. As the genome of KHV was only partially sequenced when the present inventors started their study, the insertion site of the BAC cassette was selected at the end of the TK gene **(****Fig. 3A****).** Transfection of pGEMT-TKBAC into KHV-infected CCB cells generated the KHV FL BAC strain **(****Fig. 3B****)**. The ability of the latter virus to grow in the presence of G418 allowed its isolation from the parental strain. Moreover, expression of EGFP induced by the KHV FL BAC strain was used to monitor progress of the selection process. After three passages of the virus in the presence of G418, a pure KHV FL BAC population was obtained. The molecular structure of the KHV BAC strain was confirmed by a combined *Sac*I restriction endonuclease and Southern blot approach **(****Fig. 4****).** In the KHV FL strain, the TK ORF was contained into a DNA fragment of approximately 5.2 kb. As expected, in the KHV FL BAC strain, the TK DNA fragment encompassing the BAC cassette of 9.9 kb was restricted in two fragments of approximately 5.4 kb and 9.7 kb **(****Fig. 4****)**.

Circular intermediates of the KHV FL BAC strain genome were isolated from infected cells 20 h pi and electroporated into *E. coli* DH10B cells to generate KHV FL BAC plasmid. Bacteria were immediately platted on solid LB medium to allow easier isolation of clones encoding the entire KHV genome. Indeed, it was observed that pre-amplification of bacteria in liquid led to preferential amplification of clones encoding incomplete KHV genome **(****Fig. 5****).** BAC DNAs were prepared from single colonies and screened by *Hind*III restriction endonuclease to select clones corresponding to the expected restriction profile **(****Fig. 5****)**. About five hundred clones were tested and only one clone exhibited the correct restriction profile. Indeed, as shown in **Fig. 5****,** KHV BAC clone restriction profiles obtained were very heterogeneous. Moreover, restriction profiles showed that most of BAC clones seem to have lost part of the KHV genome sequence. The molecular structure of the only complete KHV BAC plasmid obtained was characterized as described above by a combined *Sac*I restriction endonuclease and Southern blot approach **(****Fig. 4****)**. As expected, KHV FL BAC strain and KHV FL BAC plasmid profiles presented some differences due to the circularization of the KHV genome in bacteria. All together, the results presented above demonstrate that the entire KHV genome has been cloned as a BAC in *E. coli* [Deposition number LMBP 5658].

### Example 3: Stability of the KHV genome in E.coli.

BAC plasmids are usually propagated in bacteria carrying *recA* mutation that minimizes recombination. However, the large size and the complex structure of KHV genome could lead to a relative instability of the BAC plasmid (Ilouze et al., Microbiol. Mol. Biol. Rev., 70 (1), 147-156, (2006)). To assess the stability of KHV genome as a BAC plasmid, bacteria containing KHV FL BAC plasmid were serially cultured for 20 consecutive days, each day representing approximately 36 generations. After various periods of culture, the BAC plasmids were isolated and characterized by *Sac*I endonuclease digestion **(****Fig. 6****)**. No difference was observed among plasmids grown for various periods of time, demonstrating a high stability of the KHV genome in *E. coli.*

### Example 4: Reconstitution of infectious virus from the KHV FL BAC plasmid and excision of the BAC cassette from reconstituted virus.

The usefulness of a herpesvirus BAC clone requires the ability to reconstitute infectious particles from the BAC plasmid. Consequently, the inventors tested whether infectious particles could be produced by transfection of KHV FL BAC plasmid into CCB cells **(****Fig. 3B****).** Six days post-transfection, viral syncitia expressing EGFP were detected. *Sac*I restriction analysis of the DNA of reconstituted virus (KHV FL BAC recovered strain) revealed restriction profile identical to the pattern observed for KHV FL BAC strain **(****Fig. 4****).** These data demonstrated that infectious KHV FL BAC virions could be reconstituted efficiently by transfection of the KHV FL BAC plasmid into permissive cells. It is important to note that production of reconstituted virions was obtained without any promoting drug treatment. This data demonstrated that the KHV FL BAC plasmid is infectious on its own.

To excise the BAC cassette from the genome of reconstituted virions, DNA of the KHV FL BAC recovered strain was co-transfected into CCB cells with a Cre recombinase-expressing plasmid **(****Fig. 3B****)**. Deletion of the BAC cassette was monitored by the disappearance of EGFP fluorescence and by a combined restriction endonuclease and Southern blot approach **(****Fig. 4****)**. The cre-*loxP*-mediated deletion of the BAC cassette left a sequence of 172 bp at the end of TK ORF which leads to the disruption of TK ORF and thus, unable the expression of a functional TK protein (data not shown). Taken together, these data demonstrate that the KHV FL BAC plasmid is an infectious BAC clone of the KHV genome.

### Example 5: Characterization of KHV recombinant strains.

Additional characterization of KHV recombinant strains was performed. Firstly, viral syncitia generated by the KHV FL, the KHV FL BAC, the KHV FL BAC recovered and the KHV FL BAC excised strains were compared by immunofluorescent staining. The results presented in **Fig. 7A** showed that all strains were indistinguishable when revealed with anti-KHV polyclonal serum **(****Fig. 7A****, panels ii, v, viii and xi),** while EGFP expression was restricted to KHV FL BAC and KHV FL BAC recovered strains **(****Fig. 7A****, panels iv and vii).** Secondly, in order to investigate the putative effect of the recombination processes on KHV growth *in vitro,* all recombinant strains were compared using a growth assay **(****Fig. 7B****).** All viruses tested exhibit similar growth curves (P≤0.05). This latter result demonstrates not only that TK is dispensable for KHV replication *in vitro* but also that the presence of the 9.9 kb-BAC cassette inserted into the genome of the BAC strain did not affect viral replication *in vitro.*

### Example 6: BAC derived virus as vaccine.

The BAC cloning strategy of the KHV FL genome was designed to disrupt the Thymidine kinase (TK) locus. To test whether deletion of this ORF would contribute to the attenuation of viruses derived from the KHV FL BAC, the virulence of the FL BAC excised strain (which is TK -) was compared to the FL parental strain (which is TK +). This experiment was performed in 7 g koi carps **(****Fig. 8****).** This experiment demonstrated that the disruption of the TK locus contribute to a drastic attenuation of the virus. In this experiment, inoculation of 7 g koi carps with the IS, the FL and the FL BAC excised strain led to 90%, 80% and 40% mortality, respectively. Interestingly, all fishes that survived to the inoculation of the FL BAC excised strain developed a protective immune response against a further challenge with the highly virulent IS strain. All together these data support the potential of the FL BAC clone as a parental plasmid for the development of multi-attenuated recombinant vaccines.

### Example 7: KHV BAC plasmid as a parental plasmid for the production of KHV BAC recombinant plasmids by mutagenesis in bacteria using ga/K positive selection.

The results above describe the production of a TK deleted KHV strain (KHV FL BAC excised strain) that induce attenuation of KHV disease when assayed in koi carps **(****Fig. 8****).** These data support the potential of the FL BAC clone as a parental plasmid for the development of multi-attenuated recombinant vaccines. To demonstrate the feasibility of this concept, three KHV BAC recombinant plasmids, deleted for virulent gene candidates were produced by homologous recombination in bacteria using a *gal*K positive selection: a KHV BAC deleted for ORF12 encoding a putative tumor necrosis factor (TNF) receptor gene. (KHV FL BAC ΔORF12 plasmid); KHV BAC deleted for ORF16 encoding a putative G-protein coupled receptor (GPCR) gene (KHV FL BAC ΔORF16 plasmid); and a KHV BAC deleted for ORF140 encoding a putative thymidylate kinase gene (KHV FL BAC ΔORF140 plasmid). The molecular structure of the produced recombinant plasmids was confirmed by a combined *Sac*I restriction endonuclease and Southern blot approach **(****Fig. 9****).** In the parental KHV FL strain and KHV FL BAC plasmid, ORF12 and ORF16 were contained into DNA fragments of approximately 4.7 kb whereas ORF140 was contained into two DNA fragments of approximately 1.8 kb and 1 kb. As expected, in KHV FL BAC recombinant plasmid, ORF12, ORF16 and ORF140 DNA fragment encompassing the *ga*/K cassette of 1.3 kb was restricted in fragments of approximately 5.7 kb, 5 kb and 3.5 kb, respectively **(****Fig. 9****).**

These results demonstrate that the KHV FL BAC plasmid subject of the present invention can be used to produce recombinant strains using prokaryotic recombination technologies. This result was unexpected since it was hypothesized that the size of the KHV genome and its high content of repeated regions should make this process impossible due to intra-genomic recombination that should occur when turning on the expression of recombinase.

### References

Aoki, T., Hirono, I., Kurokawa, K., Fukuda, H., Nahary, R., Eldar, A., Davison, A. J., Waltzek, T. B., Bercovier, H. & Hedrick, R. P. (2007). Genome sequences of three koi herpesvirus isolates representing the expanding distribution of an emerging disease threatening koi and common carp worldwide. J Virol 81, 5058-65.
Borst, E. M., Hahn, G., Koszinowski, U. H. & Messerle, M. (1999). Cloning of the human cytomegalovirus (HCMV) genome as an infectious bacterial artificial chromosome in Escherichia coli: a new approach for construction of HCMV mutants. J Virol 73, 8320-9.
Dewals, B., Boudry, C., Gillet, L., Markine-Goriaynoff, N., de Leval, L., Haig, D. M. & Vanderplasschen, A. (2006). Cloning of the genome of Alcelaphine herpesvirus 1 as an infectious and pathogenic bacterial artificial chromosome. J Gen Virol 87, 509-17.
Gillet, L., Daix, V., Donofrio, G., Wagner, M., Koszinowski, U. H., China, B., Ackermann, M., Markine-Goriaynoff, N. & Vanderplasschen, A. (2005). Development of bovine herpesvirus 4 as an expression vector using bacterial artificial chromosome cloning. J Gen Virol 86, 907-17.
Hedrick, R. P., Gilad, O., Yun, S.C., MCdowell, T.S., Waltzek, T.B., Kelley, G.O., Adkison, M.A. (2005). Initial isolation and characterization of a herpes-like virus (KHV) from koi and common carp. Bull. Fish. Res. Agen. Supplement 2, 1-7.
Ilouze, M., Dishon, A. & Kotler, M. (2006). Characterization of a novel virus causing a lethal disease in carp and koi. Microbiol Mol Biol Rev 70, 147-56.
   Markine-Goriaynoff, N., Gillet, L., Karlsen, O. A., Haarr, L., Minner, F., Pastoret, P. P., Fukuda, M. & Vanderplasschen, A. (2004). The core 2 beta-1,6-N-acetylglucosaminyltransferase-M encoded by bovine herpesvirus 4 is not essential for virus replication despite contributing to post-translational modifications of structural proteins. J Gen Virol 85, 355-67.
Messerle, M., Crnkovic, I., Hammerschmidt, W., Ziegler, H. & Koszinowski, U. H. (1997). Cloning and mutagenesis of a herpesvirus genome as an infectious bacterial artificial chromosome. Proc Natl Acad Sci U S A 94, 14759-63.
Morgan, R. W., Cantello, J. L. & McDermott, C. H. (1990). Transfection of chicken embryo fibroblasts with Marek's disease virus DNA. Avian Dis 34, 345-51.
Neukirch, M., Böttcher, K., Bunnajrakul, S. (1999). Isolation of a virus from koi with altered gills. Bull. Eur. Ass. Fish. Pathol. 19, 221-224.
Ronen, A., Perelberg, A., Abramowitz, J., Hutoran, M., Tinman, S., Bejerano, I., Steinitz, M. & Kotler, M. (2003). Efficient vaccine against the virus causing a lethal disease in cultured Cyprinus carpio. Vaccine 21, 4677-84.
Wagner, M., Ruzsics, Z. & Koszinowski, U. H. (2002). Herpesvirus genetics has come of age. Trends Microbiol 10, 318-24.
Warming, S., Costantino, N., Court, D. L., Jenkins, N. A. & Copeland, N. G. (2005). Simple and highly efficient BAC recombineering using galK selection. Nucleic Acids Res 33, e36.

## Claims

1. A recombinant koi herpesvirus (KHV) or Cyprinid herpesvirus 3 (CyHV-3), which is immunogenic in fish, preferably in carps, more preferably in *Cyprinus carpio.*

2. The recombinant koi herpesvirus according to claim 1, which has the capability to confer immunity on fish, preferably on carps, more preferably on *Cyprinus carpio,* against a disease caused by koi herpesvirus (KHV) or Cyprinid herpesvirus 3 (CyHV-3).

3. The recombinant koi herpesvirus according to claim 1 or 2, which is deficient in one or more viral genes which is/are not essential for replication.

4. The recombinant koi herpesvirus according to any one of claims 1 to 3, which is deficient in at least one gene which contributes to virulence.

5. The recombinant koi herpesvirus according to any one of claims 1 to 4, which is deficient in at least one gene which contributes to virulence selected from the group consisting of thymidine kinase gene, ORF12: putative tumor necrosis factor (TNF) receptor gene, ORF 16: putative G-protein coupled receptor (GPCR) gene, ORF 134: putative Interleukin 10 homologue gene, ORF140: putative thymidylate kinase gene, or any combination thereof.

6. The recombinant koi herpesvirus according to any one of claims 1 to 5, wherein the herpesvirus is in a live form.

7. The recombinant koi herpesvirus according to any one of claims 1 to 5 which has the capability to reconstitute infectious particles when introduced into permissive eukaryotic cells or fish individuals.

8. The recombinant koi herpesvirus according to any one of claims 1 to 7, which herpesvirus is in an attenuated form.

9. The recombinant koi herpesvirus according to any one of claims 1 to 8, which results in a mortality rate of 40 % or less, preferably of 20 % or less, more preferred of 10 % or less, further preferred of 1% or less and most preferred of 0.1 % or less in fish, preferably in carps, more preferred *Cyprinus carpio carpio* or *Cyprinus carpio koi* infected with said herpesvirus.

10. The recombinant koi herpesvirus according to any one of claims 1 to 5, which is in inactivated and non-replicative form or in deficient attenuated form.

11. The recombinant koi herpesvirus according to any one of claims 1 to 10, comprising a BAC (bacterial artificial chromosome) vector sequence.

12. The recombinant koi herpesvirus according to any one of claims 1 to 11, wherein a BAC (bacterial artificial chromosome) vector sequence is inserted into the koi herpesvirus genome.

13. The recombinant koi herpesvirus according to claim 11 or 12, wherein the BAC vector sequence is excised from the herpesvirus genome thereby leaving a heterologous sequence at the excision site or former insertion site, respectively, in the herpesvirus genome.

14. A DNA sequence to be used for vaccine purposes and/or prophylactic health care in fish, wherein said DNA sequence which has the capability to confer immunity on fish, preferably on carps, more preferably on *Cyprinus carpio,* against a disease caused by koi herpesvirus (KHV) or Cyprinid herpesvirus 3 (CyHV-3).

15. A vector comprising a koi herpesvirus genome or a part thereof.

16. The vector of claim 15, further comprising a BAC vector sequence.

17. A cell comprising the recombinant koi herpesvirus according to any one of claims 1 to 13, or the DNA sequence according to claim 14 or the vector according to claim 15 or 16.

18. The cell of claim 17 having the deposition number LMBP 5658.

19. A virus produced by the cell of claim 18.

20. A method for the production of infectious particles of recombinant koi herpesvirus (KHV) or Cyprinid herpesvirus 3 (CyHV-3), comprising the step of introduction of any one of the following recombinant koi herpesvirus (KHV) genome or recombinant Cyprinid herpesvirus 3 (CyHV-3) genome into permissive eukaryotic cells: (a) a recombinant koi herpesvirus genome (KHV) or recombinant Cyprinid herpesvirus 3 (CyHV-3) genome comprising a bacterial artificial chromosome (BAC) vector sequence; (b) a recombinant koi herpesvirus genome (KHV) or recombinant Cyprinid herpesvirus 3 (CyHV-3) genome wherein the bacterial artificial chromosome (BAC) vector sequence is excised from the construct of (a); and culturing the host cell to produce recombinant koi herpesvirus (KHV) or recombinant Cyprinid herpesvirus 3 (CyHV-3).

21. Infectious particles generated by the method of claim 20.

22. Koi herpesvirus (KHV) or Cyprinid herpesvirus 3 (CyHV-3) deposition number LMBP 6581 CB or a derivative thereof.

23. A vaccine for preventive and/or therapeutic treatment of a disease caused by koi herpesvirus (KHV) or Cyprinid herpesvirus 3 (CyHV-3).

24. The vaccine according to claim 23 comprising the recombinant koi herpesvirus according to any one of claims 1 to 13, or the DNA sequence according to claim 14 or the vector according to claim 15 or 16 or the infectious particles according to claim 21, or comprising the koi herpesvirus (KHV) or (KHV) or Cyprinid herpesvirus 3 (CyHV-3) deposition number LMBP 6581 CB or a derivative thereof.

25. The use of the recombinant koi herpesvirus according to any one of claims 1 to 13, or the DNA sequence according to claim 14 or the vector according to claim 15 or 16 or the infectious particles according to claim 21 or the koi herpesvirus (KHV) or CyHV-3 deposition number LMBP 6581 CB or a derivative thereof for the manufacture of a medicament/vaccine for the preventive and/or therapeutic treatment of a disease caused by koi herpesvirus (KHV) or Cyprinid herpesvirus 3 (CyHV-3).

26. A method for the preventive and/or therapeutic treatment of a disease caused by koi herpesvirus (KHV) or Cyprinid herpesvirus 3 (CyHV-3) comprising the introduction of an therapeutic effective amount of the recombinant koi herpesvirus according to any one of claims 1 to 13, or the DNA sequence according to claim 14 or the vector according to claim 15 or 16 or the infectious particles according to claim 21 or the koi herpesvirus (KHV) or CyHV-3 deposition number LMBP 6581 CB or a derivative thereof into fish, preferably carps, more preferably *Cyprinus carpio carpio* or *Cyprinus carpio koi* individuals.


SEQUENCE LISTING

<110> Université de Liège

<120> A RECOMBINANT KOI HERPESVIURS (KHV) OR CYPRINID
      HERPESVIRUS 3 (CYHV-3) AND A VACCINE FOR THE PREVENTION
      OF A DISEASE CAUSED BY KHV/CYHV-3 IN CYPRINUS CARPIO
      CARPIO OR CYPRINUS CARPIO KOI

<130> P22025EP

<140> EP 07 115 093.2
<141> 2007-08-28

<160> 8

<170> PatentIn Ver. 2.1

<210> 1
<211> 21
<212> DNA
<213> Artificial Sequence

<220>
<223> Description of Artificial Sequence:
      oligonucleotide

<400> 1
atggctatgc tggaactggt g                                           21


<210> 2
<211> 20
<212> DNA
<213> Artificial Sequence

<220>
<223> Description of Artificial Sequence:
      oligonucleotide

<400> 2
ctcaacaggg aagagtggcg                                             20


<210> 3
<211> 74
<212> DNA
<213> Artificial Sequence

<220>
<223> Description of Artificial Sequence:
      oligonucleotide

<400> 3
atgaagctgc ttgtgattct gggtcttgga ctctgctacc tggctgttca cctgttgaca 60
attaatcatc ggca                                                    74


<210> 4
<211> 70
<212> DNA
<213> Artificial Sequence

<220>
<223> Description of Artificial Sequence:
      oligonucleotide

<400> 4
tcaacttctc tttggttttc tgcacatatt cgtccccccc acggttttca tcagcactgt 60
cctgctcctt                                                         70


<210> 5
<211> 74
<212> DNA
<213> Artificial Sequence

<220>
<223> Description of Artificial Sequence:
      oligonucleotide

<400> 5
atgaaacctc tgggtctttt tgtttctgtg ctcgggctgc ttgccctgtc cctgttgaca 60
attaatcatc ggca                                                    74


<210> 6
<211> 70
<212> DNA
<213> Artificial Sequence

<220>
<223> Description of Artificial Sequence:
      oligonucleotide

<400> 6

tcataggacg ccatcggttg agttcgctgc ggctgcgact cccagtcctc tcagcactgt 60
cctgctcctt 70

<210> 7
<211> 74
<212> DNA
<213> Artificial Sequence

<220>
<223> Description of Artificial Sequence:
      oligonucleotide

<400> 7
atggaacccg agacctcgac agcgagaacc gccgagcatg ccctttcatg cctgttgaca 60
attaatcatc ggca 74

<210> 8
<211> 68
<212> DNA
<213> Artificial Sequence

<220>
<223> Description of Artificial Sequence:
      oligonucleotide

<400> 8
tcaagaccat aaagtgggaa tggtgtcgtc gaagccgtct ctggagtcgt tcagcactgt 60
cctgctcc 68